# FASCICULE DE BREVET EUROPEEN

(11) **EP 1 761 240 B1**
(45) Date de publication et mention de la délivrance du brevet: **10.08.2016**
(21) Numéro de dépôt: 05751600.7
(22) Date de dépôt: 04.04.2005
(51) Int. Cl.: A61K 36/48, A61K 8/97, A61K 8/73, A61Q 17/00, A61Q 19/08, A61Q 19/00, A61K 8/64

(54) **EXTRAIT TOTAL DE LUPIN, CONSTITUE D"UN EXTRAIT DE SUCRES DE LUPIN ET D"UN EXTRAIT PEPTIDIQUE DE LUPIN, PROCEDE D"OBTENTION ET UTILISATION**
VOLLEXTRAKT AUS LUPINEN, ENTHALTEND ZUCKEREXTRAKT AUS LUPINEN UND PEPTIDEXTRAKT AUS LUPINEN, HERSTELLUNGSVERFAHREN UND VERWENDUNG
LUPIN TOTAL EXTRACT CONSISTING OF A LUPIN SUGAR EXTRACT AND A LUPIN PEPTIDE EXTRACT, METHOD FOR THE PRODUCTION AND USE THEREOF

(30) Priorité: 08.04.2004 FR 0403693
(43) Date de publication de la demande: 14.03.2007
(73) Titulaire: Laboratoires Expanscience, 92400 Courbevoie (FR)
(72) Inventeur: CHOULOT, Jean-Christophe, F-78120 Rambouillet (FR); PICCIRILLI, Antoine, F-78670 Villennes sur Seine (FR); PICCARDI, Nathalie, F-21310 Arceau (FR); MSIKA, Philippe, F-78000 Versailles (FR); PAUL, François, F-31400 Toulouse (FR)
(74) Mandataire: Regimbeau
(86) Numéro de dépôt international: PCT/FR2005/000810
(87) Numéro de publication internationale: WO 2005/102259

(56) Documents cités:
- FR-A- 2 731 351
- FR-A- 2 778 565
- US-A1- 2003 045 505
- US-A1- 2003 157 200
- DATABASE BIOSIS [Online] BIOSCIENCES INFORMATION SERVICE, PHILADELPHIA, PA, US; 1995, MOHAMED A A ET AL: "Composition of Lupinus albus" XP002308626 Database accession no. PREV199698592407 & CEREAL CHEMISTRY, vol. 72, no. 6, 1995, pages 643-647, ISSN: 0009-0352
- DATABASE BIOSIS [Online] BIOSCIENCES INFORMATION SERVICE, PHILADELPHIA, PA, US; 2003, PIOTROWICZ-CIESLAK AGNIESZKA I ET AL: "Cyclitols, galactosyl cyclitols and raffinose family oligosaccharides in Mexican wild lupin seeds." XP002308627 Database accession no. PREV200300352272 & ACTA SOCIETATIS BOTANICORUM POLONIAE, vol. 72, no. 2, 2003, pages 109-114, ISSN: 0001-6977

## Description

La présente invention concerne une composition comprenant un extrait de sucres de lupin, enrichi en galacto-oligosaccharides, et un extrait peptidique de lupin. Cette composition peut être utilisée en tant qu'agent anti-inflammatoire, réparateur de la barrière cutanée et cicatrisant, en particulier dans la prévention et/ou le traitement des érythèmes avec ou sans effraction de la barrière cutanée.

L'érythème fessier est un des troubles dermatologiques le plus fréquent des nourrissons. Il touche plus de 50% des nourrissons, avec une fréquence de 25% durant les 4 premières semaines de vie et un pic d'incidence entre 9 et 12 mois. Les irritations sont dues à des lésions de la couche cornée de l'épiderme du siège du nourrisson, qui a une double origine :
- l'activation d'enzymes urinaires et fécales stimulées par la macération sous les couches,
- l'effet abrasif dû au frottement des couches sur la peau.

Chez les bébés, l'urine n'est cependant qu'un irritant mineur de la peau. Des études réalisées chez des enfants nourris au sein ont montré que l'irritation était essentiellement due aux lipases, protéases et uréases contenues dans les selles, celles-ci étant moins actives chez les enfants nourris au sein que celles qui sont retrouvées chez les enfants nourris au biberon.

Le pH des selles de ces bébés est plus acide, à cause d'une microflore gastrique plus productive en acide métabolique. Un pH plus élevé, retrouvé dans les selles des bébés nourris au biberon, active les enzymes fécales qui sont dès lors plus agressives pour la peau. Il a été démontré que l'irritation de la peau est bien due aux enzymes fécales en chauffant les selles : celles-ci sont alors non irritantes sur la peau de l'animal ; le caractère irritant est restauré en additionnant de la trypsine et de la lipase pancréatique à ces selles.

De plus, les lipases fécales facilitent l'attaque protéolytique de la peau et augmentent sa perméabilité aux sels biliaires. Les lipases se sont pourtant révélés non irritants par eux-mêmes dans des études réalisées chez l'animal.

L'augmentation du pH est donc déterminante dans la physiopathologie de l'érythème fessier. C'est la combinaison selles-urine qui apparaît comme étant la plus dommageable. En effet, l'urée contenue dans les urines est dégradée en ammoniaque par l'uréase fécale. L'ammoniaque formée entraîne une alcalinisation du pH, qui, ainsi, potentialise l'action des enzymes fécales.

Ainsi, la formation d'ammoniac lors de la dégradation de l'urée par l'uréase joue un rôle important dans la transpiration et l'érythème fessier. Dans le cas particulier de l'érythème fessier, la formation d'ammoniac n'est pas la cause directe de l'érythème mais entraîne une augmentation de pH favorisant l'augmentation de l'activité des lipases et protéases fécales, entraînant ainsi une irritation au niveau cutané.

On connaît déjà certains agents anti-uréase contre l'érythème fessier et comme déodorant, par exemple les acides hydroxamique et hydroximique. De manière plus générale, on connaît également l'activité anti-uréase des polyhydroxyphénols et des quinones.

On connaît également l'utilisation de tanins condensés de caroube et de dérivés de l'acide chlorogénique dans des compositions cosmétiques ou dermatologiques anti-uréase. Les tanins condensés utilisés peuvent être un extrait d'Hamamélis.

Enfin, la demande de brevet FR 2 778 102 décrit l'utilisation des oligomères procyanodoliques et des anthocyanosides à titre d'agents anti-uréase.

En ce qui concerne le lupin, la demande de brevet internationale WO 00/62789 décrit l'utilisation d'un extrait peptidique de lupin dans l'inhibition des métalloprotéases. Cette demande décrit donc l'utilisation d'un extrait peptidique de lupin dans une composition destinée au traitement de l'arthrose, de maladies parodontales, de lésions de la peau, de maladies inflammatoires, ainsi que de maladies liées au défaut de cicatrisation, à l'attaque de l'émail des dents, à l'angiogénèse tumorale ou pathologique. Cette demande de brevet décrit également l'utilisation d'un tel extrait peptidique de lupin pour le traitement des lésions de la peau dues au vieillissement intrinsèque de la peau, au vieillissement sous l'action du rayonnement solaire, aux effets délétères du tabac, de la pollution et du stress.

La publication de Piotr Gulewicz et al., « Biological Activity of α-Galactoside Préparations from Lupinus angustifolius L. and Pisum sativum L. Seeds », J. Agric. Food Chem. 2002 Jan 16, 50(2) :384-9, décrit un extrait de lupin enrichi en galacto-oligosaccharides et en sucroses. Cette publication enseigne que les galacto-oligosaccharides de lupin ne sont pas cytotoxiques et qu'ils permettent l'augmentation du nombre et de la biomasse des bifidobacteria.

La demande de brevet EP 1 361 854 décrit que les activités photoprotectrices, notamment les activités anti-érythémale, anti-oxydante, anti-radicalaire, anti-inflammatoire et vasculotrope de composés de type flavonoïde, tels que la génistéine, augmentent de manière significative lorsque ledit composé est préalablement solubilisé dans un solvant approprié tel que les polyéthylène glycols, les polypropylène glycols, leurs dérivés, les alcools gras et polyols éthoxylés et leurs mélanges. Les compositions décrites dans cette demande sont particulièrement destinées à la préparation d'un médicament apte à réduite et/ou à prévenir les érythèmes, en particulier les érythèmes actiniques induits par des irradiations UVA et/ou UVB.

Malgré toutes les compositions anti-uréases proposées dans l'art antérieur, il subsiste un besoin en nouvelles compositions pour le traitement des érythèmes, en particulier de l'érythème fessier.

De manière surprenante, les inventeurs ont découvert qu'une composition comprenant un extrait total de lupin, c'est-à-dire un extrait de sucres de lupin enrichi en galacto-oligosaccharides et un extrait peptidique de lupin, pouvait être utilisée pour prévenir et/ou traiter l'inflammation cutanée et/ou des lésions de la barrière cutanée, en particulier les érythèmes.

De manière surprenante, les inventeurs ont découvert qu'une composition comprenant un extrait total de lupin est plus efficace, notamment en tant qu'agent anti-inflammatoire, qu'une composition comprenant un extrait de sucres de lupin enrichi en galacto-oligosaccharides seul ou qu'une composition comprenant un extrait peptidique de lupin seul. Il y a donc un effet de synergie entre les sucres et les peptides de lupin.

Ainsi, la présente invention a pour objet une composition comprenant un extrait total de lupin constitué d'un extrait de sucres de lupin, qui comprend au moins 50 % en poids, par rapport au poids total de la matière sèche, de galacto-oligosaccharides, et d'un extrait peptidique de lupin.

Les galacto-oligosaccharides sont des oligo-saccharides, ayant un degré de polymérisation compris entre 3 et 10, comprenant des unités galactoses.

Le lupin combine des teneurs importantes en protéines (40% en poids en moyenne) et en huile (9 à 20% en poids) comparables à celles du soja. Parmi les nombreuses variétés de lupin, le lupin blanc doux *(lupinus albus*) offre le meilleur compromis entre composition et amertume puisque, par opposition au lupin andin, le lupin blanc doux est pratiquement exsangue d'alcaloïdes.

Le lupin blanc doux comprend environ 38 à 45% en poids de protéines. Les principaux acides aminés présents dans la fraction protéique du lupin blanc sont la glutamine (20% en poids), l'asparagine et l'arginine (respectivement 15 et 10% en poids). Le lupin est toutefois pauvre en tryptophane et en acides aminés soufrés tels que la méthionine cystine et la cystéine.

Le lupin blanc comprend environ 29 à 30% en poids de carbohydrates. La fraction glucidique du lupin blanc est composé de stachyose qui est le sucre majoritaire (environ 55% en poids), suivi du saccharose (environ 25% en poids), du verbascose (environ 10% en poids) et du raffinose (environ 10% en poids). La majorité des sucres présents est constituée par des galacto-oligosaccharides.

Enfin, le lupin comprend environ 6 à 17% en poids de lipides, qui se présentent sous la forme d'une huile limpide dont on attribue la couleur jaune-orangé à la présence de provitamine A (carotènes). Cette huile est fortement insaturée et elle comprend les acides gras essentiels de type Omega 3, 6 et 9, respectivement oléique, linoléique et olinolénique.

Dans le cadre de la présente invention, l'extrait de sucres de lupin est un extrait enrichi en galacto-oligosaccharides.

Selon une variante avantageuse de l'invention, l'extrait de sucres de lupin comprend avantageusement 55 à 90% en poids, encore plus avantageusement 57 à 85% en poids, encore plus avantageusement 60 à 80% en poids, et encore plus avantageusement 65 à 75% en poids, par rapport au poids de la matière sèche dudit extrait de sucres, de galacto-oligosaccharides.

Les galacto-oligosaccharides sont avantageusement choisis dans le groupe constitué par le verbascose, le stachyose et le raffinose.

Le verbascose est un sucre en C₃₀H₅₂O₂₆, répondant à la formule suivante :

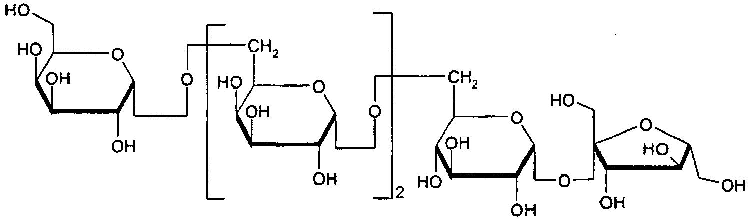

Le raffinose est un sucre en C₁₈H₃₂O₁₆, répondant à la formule suivante :

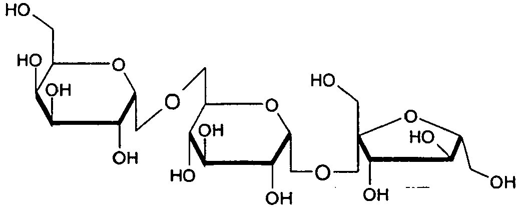

Le stachyose est un sucre en C₂₅H₄₄O₂₁, de formule générale suivante :

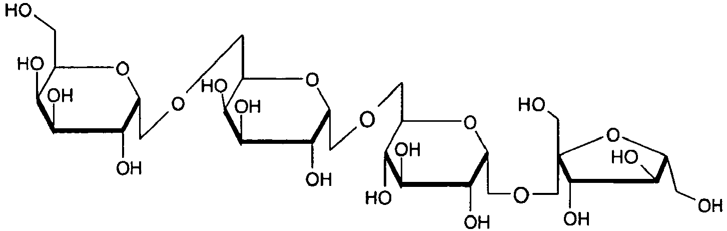

Ainsi, le stachyose, par exemple, correspond à un enchaînement de galactose-galactose-glucose-fructose, la liaison entre galactose-galactose et galactose-glucose pouvant être rompue au moyen d'une enzyme hydrolytique α-galactosidase et la liaison entre le glucose et le fructose pouvant être rompue au moyen d'une enzyme hydrolytique saccharase.

Selon une variante avantageuse de l'invention, l'extrait de sucres de lupin est susceptible d'être obtenu par un procédé comprenant les étapes suivantes :
a) extraction de lipides de la graine de lupin au moyen d'un solvant approprié et récupération des fractions protéiques et saccharidiques du lupin ; puis
b) à partir de la fraction récupérée à l'étape a), séparation de la fraction comprenant les sucres de lupin par ultrafiltration et récupération de ladite fraction saccharidique ; et
c) le cas échéant, raffinage physique de la fraction comprenant les sucres de lupin récupérée suite à l'étape b) ;
d) obtention, suite à l'étape b) ou c), d'un extrait de sucres de lupin, très enrichi en galacto-oligosaccharides.

Les lipides de la graine de lupin sont avantageusement extraits lors de l'étape a), à l'aide d'un solvant reconnu alimentaire non toxique, notamment à l'aide d'éthanol.

L'ultrafiltration est un procédé de séparation en phase liquide par perméation à travers des membranes semi-perméables sous l'action d'un gradient de pression, bien connu de l'homme du métier.

Lors de l'étape d'ultrafiltration le seuil de coupure des membranes est avantageusement de 10 000 Daltons.

Le cas échéant, la fraction comprenant les sucres de lupin obtenue suite à l'étape b) est physiquement raffinée de façon à obtenir une solution incolore et parfaitement déminéralisée, très enrichie en galacto-oligosaccharides.

Dans le cadre de la présente invention, l'extrait peptidique de lupin comprend au moins 50%, avantageusement au moins 70%, de préférence au moins 80% en poids de peptides, par rapport au poids total de l'extrait peptidique. L'extrait peptidique peut notamment être l'extrait peptidique de lupin décrit par les inventeurs dans la demande de brevet internationale WO 00/62789.

L'extrait peptidique de lupin selon l'invention est susceptible d'être obtenu par un procédé comprenant les étapes suivantes :
i) extraction des lipides de la graine de lupin au moyen d'un solvant approprié et récupération des fractions protéiques et saccharidiques du lupin ;
ii) extraction de la fraction protéique par ultrafiltration et récupération de ladite fraction protéique ;
iii) hydrolyse enzymatique des protéines, récupérées suite à l'étape ii), en peptides ; et
iv) purification par ultrafiltration des peptides obtenus suite à l'étape iii) ;
v) concentration de l'extrait obtenu suite à l'étape iv) par évaporation partielle ou totale de l'eau et récupération de l'extrait peptidique.

Le solvant approprié utilisé à l'étape i) est avantageusement choisi dans le groupe des solvants alimentaires non toxiques, notamment l'éthanol.

Selon une variante avantageuse de l'invention, l'extrait peptidique de lupin présente la composition en acides aminés donnée dans le tableau 1 suivant (pourcentage en poids par rapport au poids total d'acides aminés) :

**Tableau 1**

| aminoacides | % en poids par rapport au poids total d'acide animé |
|---|---|
| ASP | 11,3 |
| GLU | 23,2 |
| SER | 5,1 |
| HIS | 1,7 |
| GLY | 3,4 |
| THR | 3,2 |
| ALA | 2,8 |
| ARG | 10,3 |
| TIR | 6,1 |
| CYS-CYS | 2,4 |
| VAL | 3,8 |
| MET | 0,2 |
| PHE | 16,0 |
| ILE | 3,3 |
| LEU | 7,9 |
| LYS | 3,7 |
| PRO | 4,4 |

Selon une variante avantageuse de l'invention, dans l'extrait total de lupin (sucres et peptides de lupin), le rapport massique entre la matière sèche de l'extrait de sucres de lupin et la matière sèche de l'extrait peptidique de lupin est compris entre 1 et 10, avantageusement entre 1,5 et 5, encore plus avantageusement entre 2 et 3, et de préférence ce rapport est d'environ 2,5.

Selon une variante avantageuse de l'invention, dans l'extrait total de lupin (sucres et peptides de lupin), le rapport massique entre les galacto-oligosaccharides et les peptides de lupin est compris entre 1 et 10, avantageusement entre 1,5 et 5, encore plus avantageusement entre 2 et 3, et de préférence ce rapport est d'environ 2,5. Dans la graine originelle de lupin, le rapport massique entre les galacto-oligosaccharides et les peptides est d'environ 0,75.

Ainsi, dans le cadre de la présente invention, l'extrait total de lupin comprend 25 à 80% en poids de galacto-oligosaccharides, par rapport au poids de la matière sèche dudit extrait total, avantageusement 30 à 75% en poids de galacto-oligosaccharides encore plus avantageusement 33 à 68% en poids de galacto-oligosaccharides, encore plus avantageusement 40 à 60% en poids de galacto-oligosaccharides, encore plus avantageusement 43 à 53% en poids de galacto-oligosaccharides, encore plus avantageusement 45 à 51 % en poids de galacto-oligosaccharides, par rapport au poids de la matière sèche de l'extrait total de lupin.

Dans le cadre de la présente invention, le lupin est avantageusement choisi dans le groupe constitué par les variétés *lupinus angustifolius, lupinus albus, lupinus luteus, lupinus mutabilis,* de préférence le lupin est du genre *lupinus albus.* Comme exemple de lupin du genre lupin blanc doux (*lupinus albus*), on peut notamment citer la variété Ares, qui présente un faible teneur en alcaloïdes.

La composition selon l'invention peut en outre comprendre, d'une manière facultative, un dérivé de chromane ou de chromène répondant à la formule générale (I). 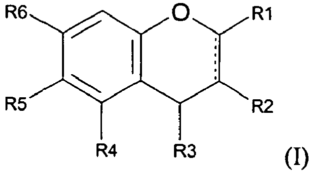 dans laquelle :
la ligne en pointillés représente une liaison additionnelle ou un défaut de liaison additionnelle ;
R₁ représente un atome d'hydrogène, un radical hydroxy, un radical méthoxy, un radical phényle, un radical phényle substitué par 1, 2 ou 3 groupes hydroxy, un radical phényle substitué par 1, 2 ou 3 groupes méthoxy ou un radical phényle substitué par un radical flavone de formule : 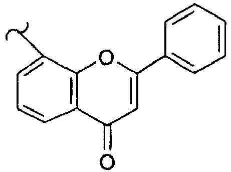
R₂ représente un atome d'hydrogène, un radical hydroxy, un radical méthoxy, un radical phényle, un radical phényle substitué par 1, 2 ou 3 groupes hydroxy ou un radical phényle substitué par 1, 2 ou 3 groupes méthoxy
   ou bien R₁ et R₂ forment ensemble un cycle benzénique ;
R₃ représente un atome d'hydrogène, un radical hydroxy ou un radical oxo ; R₄, R₅ et R₆, identiques ou différents, représentent un atome d'hydrogène, un radical hydroxy, un radical méthoxy, un radical phényle, un radical phényle substitué par 1, 2 ou 3 groupes hydroxy ou un radical phényle substitué par 1, 2 ou 3 groupes méthoxy.

Dans un mode de réalisation particulier de la présente invention, le dérivé de chromane ou de chromène est choisi dans le groupe constitué par les chromones, les xanthones et les flavonoïdes. Selon la présente invention, le dérivé de chromane ou de chromène est avantageusement un flavonoïde.

Par le terme de « flavonoïde », on entend, au sens de la présente invention, des composés comprenant deux cycles aromatiques liés entre eux par trois atomes de carbone qui forment comme un hétérocyle oxygéné. Selon la présente invention, le flavonoïde est avantageusement choisi dans le groupe constitué par les flavones, les flavonols, les dihydro-2,3-flavonols, les flavanones, les flavanols, les flavandiols, les isoflavonoïdes et les biflavonoïdes.

Par le terme de « flavanol », on entend, au sens de la présente invention, les hydroxy-3-flavanes ou les hydroxy-4-flavanes.

Par le terme de « flavane », on entend, au sens de la présente invention, les composés de formule :

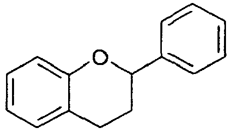

Par le terme de « flavandiol », on entend, au sens de la présente invention, les dihydroxy-3,4-flavanes.

Par le terme de « isoflavonoïde », on entend, au sens de la présente invention, les isoflavones, ainsi que les isoflavones de formule développée 3-phényl-4-oxo-dihydro-2,3-chromènes.

Par le terme de « biflavonoïde », on entend, au sens de la présente invention, des composés de formule :

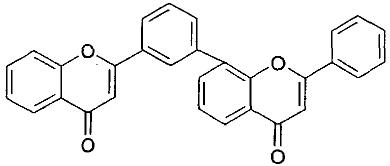

Selon la présente invention, le flavonoïde est avantageusement une isoflavone ou un mélange d'isoflavones. Les isoflavones utilisables selon la présente invention sont obtenues par synthèse chimique ou sont des substances naturelles extraites de produits naturels, notamment à partir de végétaux. On distingue des formes aglycones, des isoflavones et les formes glycosylées de ces dernières. Ces diverses formes sont illustrées par les formules suivantes.

Formes aglycones de formule : 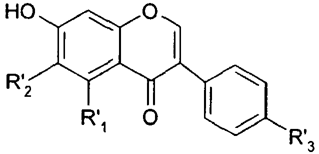 dans laquelle R'₁ représente un atome d'hydrogène ou un groupe hydroxy, R'₂ représente un atome d'hydrogène ou un groupe méthoxy et R'₃ représente un groupe hydroxy.

Avantageusement, selon la présente invention, R'₁, R'₂ et R'₃ représentent :

| R'₁ | R'₂ | R'₃ | Nom du composé |
|---|---|---|---|
| H | H | OH | Daidzéine |
| OH | H | OH | Génistéine |
| H | OCH₃ | OH | Glycitéine |

Formes glycosylées de formule : 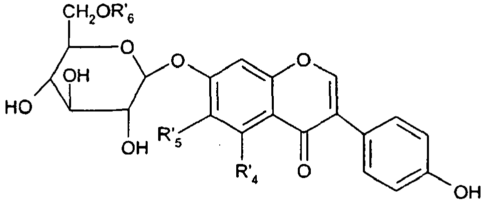 dans laquelle R'₄ représente un atome d'hydrogène ou un groupe hydroxy, R'₅ représente un atome d'hydrogène ou un groupe méthoxy et R'₆ représente un atome d'hydrogène.

Avantageusement, selon la présente invention, R'₄, R'₅ et R'₆ représentent :

| R'₄ | R'₅ | R'₆ | Nom du composé |
|---|---|---|---|
| H | H | H | Daidzine |
| OH | H | H | Génistine |
| H | OCH₃ | H | Glycitine |

Les formes glycosylées des isoflavones sont les plus abondantes dans la nature. Cependant, les isoflavones aglycones présentent des activités biologiques nettement supérieures, à leurs homologues glycosylés. Tel est le cas des isoflavones naturelles telles que la génistéine, la daidzéine ou la glycitéine.

L'isoflavone selon la présente invention est avantageusement choisie dans le groupe constitué par la génistéine, la daidzéine et la glycitéine, avantageusement la flavonoïde est la génistéine. En particulier, la génistéine utilisable selon la présente invention est un produit d'origine végétale titrant de 85 à 90% en poids.

La présente invention a également pour objet une composition cosmétique et/ou pharmaceutique comprenant une composition telle que définie précédemment, c'est-à-dire comprenant un extrait total de lupin, (extrait de sucres de lupin et extrait peptidique de lupin), et en outre, éventuellement, un dérivé de chromane ou de chromène.

La composition cosmétique et/ou pharmaceutique est avantageusement destinée à un usage externe topique, elle peut par ailleurs contenir un support cosmétiquement et/ou pharmaceutiquement acceptable.

La composition cosmétique et/ou pharmaceutique, qui est mise en oeuvre avantageusement selon la présente invention, peut se présenter sous toutes les formes galéniques usuellement utilisées pour une application externe topique. Selon la présente invention, la composition se présente avantageusement sous forme d'une solution aqueuse, hydroalcoolique ou huileuse, d'une émulsion huile-dans-eau ou eau-dans-huile ou multiple, d'un gel aqueux ou huileux, d'un produit anhydre liquide, pâteux ou solide, ou d'une dispersion d'huile dans une phase aqueuse à l'aide de sphérules, les sphérules pouvant être des nanoparticules polymériques, telles que les nanosphères et les nanocapsules, ou des vésicules lipidiques de type ionique et/ou non-ionique. La composition cosmétique peut être plus ou moins fluide, se présenter sous forme de crème blanche ou colorée, de pommade, de lait, de lotion, d'onguent, de sérum, de pâte, de mousse, d'aérosol ou de stick.

La composition cosmétique et/ou pharmaceutique mise en oeuvre selon la présente invention peut par ailleurs contenir les adjuvants habituels dans le domaine cosmétique et/ou pharmaceutique, tels que les gélifiants hydrophiles ou lipophiles, les actifs hydrophiles ou lipophiles, les conservateurs, les anti-oxydants, les solvants, les parfums, les charges, les filtres chimiques ou minéraux, les pigments, les agents chélateurs, les absorbeurs d'odeur, les eaux thermales et les matières colorantes. Les quantités de ces différents adjuvants sont celles classiquement utilisées en cosmétique et/ou pharmaceutique, et par exemple de 0,01% à 20% en poids, par rapport au poids total des compositions cosmétiques et/ou pharmaceutiques. Ces adjuvants, selon leur nature, peuvent être introduits dans la phase grasse, dans la phase aqueuse, dans les vésicules lipidiques et/ou dans les nanoparticules.

Lorsque la composition selon la présente invention est une émulsion, la proportion de la phase grasse peut aller de 5% à 80% en poids, de préférence de 5% à 50% en poids, par rapport au poids total de la composition cosmétique et/ou pharmaceutique. Les huiles, les émulsionnants et les co-émulsionnants utilisés dans la composition cosmétique et/ou pharmaceutique sous forme d'émulsion sont choisis parmi ceux classiquement utilisés dan leur domaine considéré. L'émulsionnant et le co-émulsionnant sont présents dans la composition en une proportion allant de 0,3% à 30% en poids, de préférence de 0,5% à 20% en poids, par rapport au poids total de la composition cosmétique.

Parmi les huiles utilisables selon la présente invention, on peut citer notamment les huiles minérales, d'autres huiles d'originale végétale (huile d'abricot, de tournesol, de prune), les huiles d'origine animale, les huiles de synthèse, les huiles siliconées et les huiles fluorées (perfluoropolyéthers). Des alcools gras, tels que l'alcool cétylique, des acides gras ou des cires, telles que la cire d'abeille, peuvent également être utilisées en tant que matières grasses selon la présente invention.

Parmi les émulsionnants et coémulsionnants utilisables selon la présente invention, on peut citer notamment les esters d'acide gras et de polyéthylène glycol, tels que le stéarate de PEG-40 ou le stéarate de PEG-100, les esters d'acide gras et de polyol, tels que le stéarate de glycérol et le tristéarate de sorbitan.

Parmi les gélifiants hydrophiles utilisables selon la présente invention, peuvent notamment être cités les polymères carboxyvinyliques (carbomère), les copolymères acryliques tels que les copolymères d'acrylate/alkylacrylate, les polyacrylamides, les polysaccharides, les gommes naturelles et les argiles. Parmi les gélifiants lipophiles, peuvent notamment être cités les argiles modifiées telles que les bentones, les sels métalliques d'acides gras, la silice hydrophobe et les polyéthylènes.

Les compositions cosmétiques et/ou pharmaceutiques selon l'invention peuvent également être formulées comme produits d'hygiène corporelle, notamment d'hygiène intime. On peut citer de façon non limitative les produits anti-transpirants, déodorants sous forme de spray ou gel, les shampooings, notamment anti-pelliculaires, les savons et les produits d'hygiène dentaire,

La présente invention a également pour objet l'utilisation d'une composition comprenant un extrait de sucres de lupin, qui comprend au moins 50% en poids, par rapport au poids total de la matière sèche, avantageusement de 55 à 90% en poids, encore plus avantageusement de 57 à 85% en poids, encore plus avantageusement de 60 à 80% en poids, et encore plus avantageusement de 65 à 75% en poids, par rapport au poids total de la matière sèche, de galacto-oligosaccharides, en tant qu'agent anti-inflammatoire.

En effet, les inventeurs ont découvert que l'extrait de sucres de lupin selon la présente invention présente une action anti-inflammatoire. Il peut donc être utilisé dans la fabrication d'un médicament, notamment destiné au traitement ou à la prévention des réactions ou pathologies allergiques, inflammatoires et/ou irritatives de la peau et/ou des muqueuses. En particulier, les pathologies inflammatoires, peuvent être des dermatoses inflammatoires telles que le psoriasis, des dermites irritatives, des maladies auto-immunes, une photo-immuno-suppression ou un rejet de greffe. Par «photo-immuno-suppression » au sens de la présente invention, on entend désigner une diminution de la réponse immunitaire induite par les ultra-violets solaires, et plus particulièrement par les ultra-violets B.

Selon un mode de réalisation de la présente invention, le médicament comprenant un extrait de sucres de lupin est destiné au traitement ou à la prévention des peaux et/ou des muqueuses sensibles, réactives, inconfortables, intolérantes, présentant un trouble de la barrière cutanée et/ou présentant un déséquilibre immunologique lié au vieillissement intrinsèque, extrinsèque (soleil, pollution) ou hormonal.

Selon une autre variante de l'invention, l'extrait de sucres de lupin est également utilisé pour diminuer le caractère allergisant et/ou irritant d'une composition telle qu'une préparation pharmaceutique, une préparation cosmétique ou un parfum. Par caractère allergisant, on entend le pouvoir de certains composés contenus dans les dites compositions de se comporter comme des allergènes, c'est-à-dire des composés capables d'induire une réaction d'hypersensibilité immédiate et/ou inflammatoire.

La présente invention concerne également une méthode de traitement cosmétique des peaux et/ou des muqueuses sensibles, irritées, intolérantes, à tendance allergique, âgées, présentant un trouble de la barrière cutanée, présentant des rougeurs cutanées, ou présentant un désordre ou un déséquilibre immunologique non pathologique lié au vieillissement intrinsèque, extrinsèque ou hormonal, caractérisée en ce qu'elle consiste à appliquer sur la peau et/ou les muqueuses un extrait de sucres du lupin ou une composition cosmétique comprenant ledit extrait.

La présente invention concerne également l'utilisation d'une composition comprenant un extrait total de lupin, (extrait de sucres de lupin et extrait peptidique de lupin), et en outre, éventuellement, un dérivé de chromane ou de chromène, pour la fabrication d'un médicament. Le médicament est notamment destiné à la prévention et/ou au traitement des érythèmes, en particulier des érythèmes fessiers ou des érythèmes solaires, de la couperose, ainsi que toute inflammation cutanée consécutive :
i) à une agression externe ; et/ou
ii) à un dysfonctionnement du métabolisme ou de la structure de la peau.

Dans le cadre de la présente invention, on entend par l'expression « dysfonctionnement du métabolisme ou de la structure de la peau », le terme « peau » représentant la peau kératinisée ou des muqueuses orales, gingivales, nasales ou vaginales, toutes les agressions, les déséquilibres, les anomalies de la barrière cutanée, entraînant un inconfort ou une pathologie, qui peuvent être aggravés par un événement externe ou des facteurs endogènes: sécheresse, ichtyose, ulcères...De même on entend par ladite expression toutes les maladies inflammatoires cutanées provoquant ou siégeant sur une barrière cutanée dégradée : atopie, psoriasis, dermite séborrhéique, acnée, erythro-couperose ou rosacée ainsi que les maladies ou dysfonctionnements faisant le lit ou étant provoquées par des germes pathogènes ou non. Cette énumération de pathologies n'a pas de caractère limitatif car bien souvent altération de la barrière inflammation et prolifération bactérienne ou fongique sont liées.

Dans le cadre de la présente invention, l'agression externe peut être une agression induite par des irradiations UVA et/ou UVB. Dans le cas d'un érythème solaire, les compositions cosmétiques ou les médicaments selon la présente invention sont appliqués directement sur la peau avant et/ou pendant et/ou après exposition au soleil, et avantageusement après l'apparition d'un érythème sur la peau. L'agression externe peut aussi être toutes agressions météorologiques (froid, vent) et tout contact avec un irritant, un allergène chimique ou naturel ou un agent rubéfiant. Il peut s'agir également des frottements répétitifs des couches de l'enfant, de la femme ou du vieillard mais aussi consécutif à un geste médical ou chirurgical (laser, peeling, épilation électrique, électrocoagulation). De même la macération des plis et des aisselles ou des muqueuses optimise par l'occlusion la prolifération des germes et l'inflammation.

La composition selon l'invention, comprenant un extrait total de lupin, présente une triple activité anti-enzymatique, c'est-à-dire une activité anti-protéase, une activité anti-lipase et une activité anti-uréase combinées.

Ainsi, la composition selon l'invention peut être utilisée pour la fabrication d'un médicament destiné à la prévention et/ou au traitement des réactions ou pathologies allergiques, inflammatoires et/ou irritatives de la peau et/ou des muqueuses. La composition selon l'invention peut également être utilisée comme agent pour favoriser la cicatrisation.

La présente invention concerne également une méthode de traitement cosmétique des peaux et/ou des muqueuses sensibles, irritées, intolérantes, à tendance allergique, âgées, présentant un trouble de la barrière cutanée, présentant des rougeurs cutanées, ou présentant un désordre ou un déséquilibre immunologique non pathologique lié au vieillissement intrinsèque, extrinsèque ou hormonal, caractérisée en ce qu'elle consiste à appliquer sur la peau et/ou les muqueuses un extrait total de lupin ou une composition cosmétique comprenant ledit extrait.

La présente invention concerne aussi une méthode de traitement cosmétique pour réguler le pH de la peau, caractérisée en ce qu'elle consiste à appliquer sur la peau et/ou les muqueuses un extrait total de lupin ou une composition cosmétique comprenant ledit extrait.

La présente invention concerne enfin une méthode de traitement cosmétique pour réparer l'état de surface de la peau, notamment pour réparer des lésions de la barrière cutanée, caractérisée en ce qu'elle consiste à appliquer sur la peau un extrait total de lupin ou une composition cosmétique comprenant ledit extrait.

Lorsque la peau est soumise
i) à une agression externe ; et/ou
ii) à un dysfonctionnement de son métabolisme ou de sa structure,
sa surface peut être altéreé et/ou on peut observer des lésions de la barrière cutanée.

Les exemples suivants sont donnés à titre non limitatif et illustrent la présente invention.
La figure 1 représente la quantité d'IL-1α relargué par les kératinocytes, en fonction du temps (cinétique de relarguage).
La figure 2 représente l'activité anti-uréase de la formule exemplifiée à l'exemple 3, illustrée par la valeur du pH en fonction du temps lors de l'ajout d'une solution d'uréase.
La figure 3 représente le pourcentage d'inhibition de l'activité lipase par rapport à un témoin pour une solution testée plus ou moins diluée. Les valeurs données (1/1200, 1/120, 1/48 et 1/12) correspondent aux dilutions de la solution testée (v/v).

### Exemple 1 : Composition d'un extrait total de lupin selon l'invention

**Tableau 2**

| **Critères de composition** | |
|---|---|
| Aspect | Solution aqueuse de couleur jaune pâle |
| Teneur en matière sèche (MS) | 35 % |
| Teneur en peptides totaux | 10% |
| % azote alpha-aminé (équivalent leucine/MS) | 5,5 |
| Teneur en galacto-oligosaccharides de lupin (% HPLC / MS) | 48% |
| pH | 6,5 |
| Absorbance à 420 nm | 0,420 |
| Absorbance à 550 nm | 0,024 |
| Conservateur | 0,45 % |

La matière sèche est constituée des peptides et des sucres de lupin, ainsi l'extrait total de lupin comprend 25% en poids de sucres, par rapport au poids total de l'extrait. Ainsi, dans cet extrait total de lupin, le rapport extrait de sucres de lupin sur extrait peptidique de lupin est de 2,5. Cet extrait total de lupin comprend 48% en poids de galacto-oligosaccharides, par rapport au poids total de la matière sèche dudit extrait total. Ainsi, l'extrait de sucres de lupin comprend 67,2 % en poids de galacto-oligosaccharides, par rapport au poids total de la matière sèche dudit extrait de sucres.

### Exemple 2: procédé de préparation d'un extrait total de lupin selon l'invention

### A) Procédé de préparation d'un extrait de sucres de lupin a) Délipidation à l'éthanol

On mélange 9,9 kg farine de lupin à 85 L d'éthanol et on maintient ce mélange sous agitation 1h à température ambiante. Ensuite, on laisse le mélange se décanter puis on élimine la fraction alcoolique.

### b) Récupération de la fraction saccharidique :

### i) Dispersion dans l'eau et ajustement du pH

On ajoute 90 L d'eau à la fraction décantée puis on ajustement le pH à 8,5 à l'aide de soude.

### ii) Centrifugation et filtration avec un filtre à plaques

La solution ainsi obtenue est centrifugée sur décanteur continu type Westfalia, puis filtrée sur filtre à plaques. On récupère le filtrat.

### iii) Ultrafiltration avec une membrane de seuil de coupure 10kDa et concentration

Le filtrat est concentré par un facteur 2. On effectue ensuite une diafiltration par 2 volumes d'eau déminéralisée (soit 140 L d'eau déminéralisée) et on récupère l'ultrafiltrat, qui est ensuite concentré avec une membrane de seuil de coupure 200 Da (facteur de concentration égal à 8), soit un volume total de sucres concentrés de 15 L.

### c) Raffinage physique de la fraction saccharidique :

### i) Déminéralisation et concentration

Les sucres concentrés obtenus passent à travers 2 colonnes de résines échangeuses d'ions montées en série (échangeuse de cations et échangeuse d'anions respectivement) puis ils sont concentrés par nanofiltration (membrane de seuil de coupure 200 Da). On récupère des sucres déminéralisés concentrés (4 L à 17 g/L de sucres).

### ii) Décoloration au charbon actif

0,1 kg de charbon est ajouté aux 4 L de solution de sucres. On laisse agir 1 heure à température ambiante puis on filtre avec un filtre à plaques.

### iii) Concentration finale

Le filtrat récupéré est concentré par nanofiltration avec une membrane de seuil de coupure 200 Da. Au final, on obtient 2,46 kg de solution de sucres à 275 g/L.

### B) Procédé de préparation d'un extrait peptidique de lupin

### a) Extraction et purification des protéines de lupin

Cette étape comprend une solubilisation aqueuse de la fraction soluble à pH alcalin suivie d'une séparation des insolubles :
A partir du tourteau de lupin délipidé et broyé, l'extraction des protéines est réalisée à pH 9,0 (pH ajusté par ajout de soude) avec un ratio farine/eau égal à 1/10 (p/p). La solution est incubée sous agitation à température ambiante pendant une heure. La partie insoluble du tourteau est ensuite séparée de la partie soluble par essorage. Le gâteau obtenu est lavé. La fraction soluble contenant les protéines et les sucres solubles est diafiltrée sur un module d'ultrafiltration de seuil de coupure de 10 000 daltons afin de séparer les protéines(rétentat) des sucres solubles (ultrafiltrat).

### b) Production et purification de peptides par hydrolyse enzymatique:

Le rétentat d'ultrafiltration contenant les protéines est ajusté à la concentration de 100 g/l puis hydrolyse à pH 8,0 en présence de glucanase à 55°C pendant 3 heures environ. Après hydrolyse, l'enzyme est dénaturée par traitement thermique pendant 15 min à 85°C. Dès que la solution est refroidie, elle est neutralisée par ajout d'acide chlorhydrique. Les peptides obtenus sont purifiés par diafiltration sur module d'ultrafiltration de seuil de coupure de 10 000 daltons. La solution obtenue est ensuite nanofiltrée afin de dessaler (élimination du chlorure de sodium) et de concentrer la fraction peptidique. La solution de peptides est enfin décolorée àl'aide de charbon actif 3 % (1 heure à 50°C), le charbon étant éliminé par filtration.

La solution concentrée à deshydrater est ensuite injectée sous forme d'un fin brouillard en haut d'une tour en même temps qu'un courant d'air chaud. Enfin, la poudre est récupérée, séchée par atomisation puis conditionnée.

### C) Réalisation de l'Extrait Total de Lupin

On mélange l'extrait peptidique de lupin, l'extrait de sucres de lupin et le conservateur. Ainsi, sous agitation, on mélange :
- 2,46 kg de sucres de lupin à 275 g/L MS soit 0,676 Kg de MS ;
- 0,32 kg d'extrait peptidique de lupin à 95 % de MS, soit 0,304 Kg de MS ; et
- 12,5 g de conservateur (0,4% p/p).

On a alors un rapport massique MS sucres de lupin / MS extrait peptidique de lupin de 2,22.

On homogénéise ensuite sous agitation mécanique puis on effectue une microfiltration stérilisante et un conditionnement :
- Filtration en 2 étapes : filtre à plaques puis en conditions stériles avec une cartouche stérilisante de seuil de coupure 0,2 µm ;
- Conditionnement.
Quantité produite : 2,6 kg à 36,2 % de MS.
Le tableau 3 suivant donne la composition de l'extrait total de lupin préparé suivant le procédé décrit ci-dessus :

**Tableau 3**

| Aspect | Solution légèrement orangée | |
|---|---|---|
| *Critères analytiques* | | |
| Matière sèche (MS) | **36,2 %** | |
| pH (dilution ¼) | 6,69 | |
| Absorbance à 420 nm | 0,6 | |
| Absorbance à 550 nm | 0,053 | |
| *Composition de la matière sèche* | | |
| Azote α - aminé | 5,1 % P/P de MS | |

| | % en poids / poids de l'extrait total | % en poids / poids de MS de l'extrait total |
|---|---|---|
| Monosaccharides | 3,6 | 10 |
| Saccharose | 4 | 11 |
| Stachyose | 12,9 | 35,6 |
| Verbascose | 2 | 5,5 |
| Raffinose | 2,5 | 6,9 |
| Total sucres | 25 | **69** |
| Total galacto-oligosaccharides | 17,4 | **48** |

**Exemple 3: Formulation destinée au traitement et/ou à la prévention de l'érythème fessier :**

| | |
|---|---|
| Eau purifiée | 56,6 % |
| Oxyde de zinc | 10,0 % |
| Caprylyl glycol | 5,0 % |
| Huile vaseline fluide | 5,0 % |
| Dioctanoate de propylène glycol | 4,0 % |
| Méthyl glucose dioléate | 4,0 % |
| Oxyde de titane organophile | 3,0 % |
| PEG-45/dodécyl glycol copolymère | 3,0 % |
| Cire de cérésine | 2,0 % |
| **Extrait total de lupin** | **2,0 %** |
| Penthanol dextrogyre | 2,0 % |
| Vitamine F neutre à 35% | 1,0 % |
| Beurre de karité | 1,0 % |
| Sorbate de potassium | 0,5 % |
| Parahydroxybenzoate de méthyle | 0,3 % |
| Sulfate de magnésium | 0,3 % |
| Parahydroxybenzoate de propyle | 0,2 % |
| **Génistéine** | **0,10 %** |

**Exemple 4: Formulation pour une crème lavante hygiène intime**

| **Ingrédients** | **% w/w** |
|---|---|
| Eau | QSP 100 |
| Coco-Glucoside | 10,00 |
| Disodium Lauryl Sulphosuccinate | 2,00 |
| Sodium Cocoyl Iséthionate | 2,00 |
| Amidon de maïs | 2,00 |
| Alcool Cetéarylique | 2,00 |
| Hydroxypropyl Guar | 2,00 |
| Acide citrique | QS pH |
| Extrait Total de Lupin | 0,1 à 10 |
| Génistéine | 0,1 à 10 |
| Glycérine | 0,45 |
| Polyquaternium 10 | 0,30 |
| Tétrasodium EDTA (acide éthylène diamine tétraacétique) | 0,20 |
| Dioxyde de Titane | 0,10 |
| Conservateur | QS |

**Exemple 5: Formulation pour un moussant hygiène intime**

| **Ingrédients** | **% w/w** |
|---|---|
| Eau | QSP 100 |
| Sodium Lauroamphoacétate | 10,00 |
| Coco-Glucoside | 10,00 |
| Magnésium Laureth Sulfate | 5,00 |
| PEG-40 Glycéryl Cocoate | 2,50 |
| PEG-150 Distéarate | 1,00 |
| Sodium Coceth Sulfate | 1,00 |
| Extrait Total de Lupin | 0,1 à 10 |
| Génistéine | 0,1 à 10 |
| Acide Citrique | QS pH |
| Parfum | QS |
| Conservateurs | QS |

**Exemple 6: Formulation destinée au traitement et/ou à la prévention de l'érythème fessier :**

| | |
|---|---|
| Eau purifiée | 56,7 % |
| Oxyde de zinc | 10,0 % |
| Caprylyl glycol | 5,0 % |
| Huile vaseline fluide | 5,0 % |
| Dioctanoate de propylène glycol | 4,0 % |
| Méthyl glucose dioléate | 4,0 % |
| Oxyde de titane organophile | 3,0 % |
| PEG-45/dodécyl glycol copolymère | 3,0 % |
| Cire de cérésine | 2,0 % |
| Extrait total de lupin | 2,0 % |
| Penthanol dextrogyre | 2,0 % |
| Vitamine F neutre à 35% | 1,0 % |
| Beurre de karité | 1,0 % |
| Sorbate de potassium | 0,5 % |
| Parahydroxybenzoate de méthyle | 0,3 % |
| Sulfate de magnésium | 0,3 % |
| Parahydroxybenzoate de propyle | 0,2 % |

### Exemple 7: Activité anti-inflammatoire des sucres de lupin

Une inflammation a été modélisée *in vitro* par l'addition de SDS (sodium dodécyl sulfate). Le SDS induit une production d'IL-1 bêta, médiateur clé de la réaction inflammatoire, par les kératinocytes. La quantité d'IL-1 bêta produite par les kératinocytes en réponse au SDS est donc proportionnelle à l'intensité de la réponse inflammatoire.

Une cytoxicité cellulaire a été préalablement réalisée (test au rouge neutre) afin de déterminer des doses de travail non toxiques pour les cellules. Ainsi, la concentration inhibitrice 50 IC₅₀ (concentration qui induit une mortalité de 50% des cellules, par rapport au nombre total de cellules) est d'environ 10% de l'extrait de sucres de lupin à 24 heures et 48 heures.

Les cellules sont pré-traitées pendant 24 heures avec différentes concentrations d'une solution d'extrait de sucres de lupin (0,5% ; 1% et 5%, soit 0,375% ; 0,75% et 3,75% de galacto-oligosaccharides), puis sont mises en présence de SDS (20 µg/ml) pendant 16 heures. La quantité d'IL-1 bêta est dosée dans les milieux de culture par un test ELISA (dosage immunologique).

Le tableau 4 suivant représente les résultats obtenus.

**Tableau 4**

| | Cellules contrôles | SDS | 0,5% d'extrait de sucres de lupin | 1% d'extrait de sucres de lupin | 5% d'extrait de sucres de lupin |
|---|---|---|---|---|---|
| IL-1 bêta (pg/ml) | 0,26 | 10,42 | 5,25 | 6,33 | 3,83 |

Ainsi, les sucres de lupin diminuent de 50 à 60% (pour la plus forte concentration utilisée soit 5%) la production d'IL-1 bêta par des kératinocytes soumis à un stress inflammatoire. Les sucres de lupin possèdent donc une activité anti-inflammatoire très significative.

### Exemple 8: Activité anti-inflammatoire de l'extrait total de lupin (sucres de lupin enrichis en galacto-oligosaccharides et peptides de lupin)

Le protocole expérimental est identique à celui de l'exemple 7. Les résultats sont exprimés en pourcentage d'inhibition de la synthèse d'IL-1 béta par des cellules traitées (sucres + peptides + SDS) par rapport à des cellules non traitées (SDS seul).

**Tableau 5**

| | | | | | | | |
|---|---|---|---|---|---|---|---|
| Pourcentage de galactooligosaccharides | 0,375 | 0,75 | 3,75 | 0,01 | 0,375 | 0,75 | 3,75 |
| Pourcentage de peptides | 0 | 0 | 0 | 0 | 0,01 | 0,01 | 0,01 |
| % d'inhibition | 49 | 39 | 63 | 18 | 90 | 88,5 | 95 |

Les peptides de lupin seuls présentent donc une activité anti-inflammatoire modérée. De manière tout à fait inattendue, lorsque les peptides et les sucres de lupin sont utilisés simultanément, l'activité anti-inflammatoire des sucres est potentialisée, et vice versa. Les sucres et les peptides de lupin agissent donc en synergie pour inhiber l'inflammation induite par le SDS.

### Exemple 9: Activité anti-inflammatoire d'une composition comprenant un extrait total de lupin (sucres de lupin enrichis en galacto-oligosaccharides et peptides de lupin) et un dérivé de chromène ou de chromane

On a testé la formule exemplifiée à l'exemple 3.

Une inflammation a été modélisée *in vitro,* sur un modèle d'épiderme reconstruit (Skinethic®) exposé à l'action du SLS (sodium lauryl sulfate). La quantité d'IL-1α, médiateur majeur des phénomènes irritatifs et inflammatoires relargué par les kératinocytes, a été quantifiée à plusieurs temps (cinétique de relarguage).

La formule a été appliquée sur les épidermes soit avant l'ajout de SLS (évaluation de l'effet préventif), soit après l'ajout de SLS (évaluation de l'effet curatif).

Le SLS induit une production d'IL-1α de façon temps-dépendant. Durant les 8 premières heures, la formule testée diminue d'environ 40% la production et/ou le relargage de l'IL-1α, avec une efficacité comparable en prévention et curatif.

Au-delà de 8 heures, l'effet curatif (50% d'inhibition) semble supérieur à l'effet préventif (30% d'inhibition).

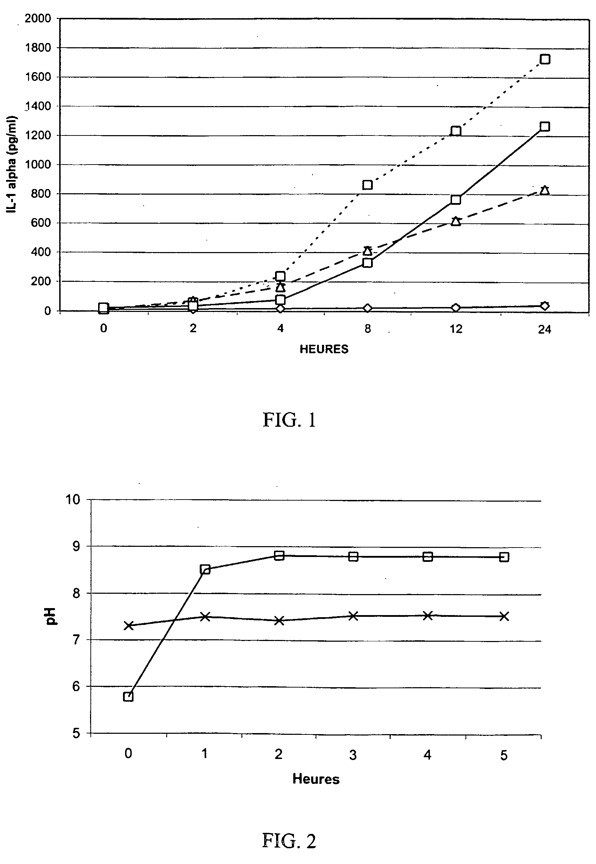

La Figure 1 ci-jointe représente les résultats obtenus (production de IL- 1α (pg/ml) en fonction du temps (heures)).

**Légende:**

| | |
|---|---|
| -◊- | Contrôle |
| ----□---- | SLS seul |
| ---Δ--- | SLS puis formule testée |
| -□- | Formule testée puis SLS |

En conclusion, ce test démontre que la formule permet de limiter la réaction inflammatoire induite par le SLS dans un modèle d'épiderme reconstruit. Son action est à la fois curative et préventive.

### Exemple 10: Activité anti-uréase d'une composition comprenant un extrait total de lupin (sucres de lupin enrichis en galacto-oligosaccharides et peptides de lupin) et un dérivé de chromène ou de chromane

On a testé la formule exemplifiée à l'exemple 3. Le protocole est basé sur la mesure du pH. Dans une fiole, on introduit le produit à tester, on ajoute un tampon pH = 6 puis on ajoute une solution d'urée. Ensuite, on ajoute une solution d'uréase et on mesure le pH de la phase aqueuse toutes les heures. L'urée va se dégrader en ammoniaque en présence d'uréase et le pH va augmenter. Une activité anti-uréase se traduira par une stabilisation du pH. La Figure 2 ci-jointe représente les résultats obtenus (pH en fonction du temps exprimé en heures).

**Légende :**

| | |
|---|---|
| -□- | Témoin |
| -×- | Formule selon l'invention |

Le témoin correspond à une solution d'urée et d'uréase sans produit actif. L'uréase va dégrader l'urée en ammoniaque.
L'activité anti-uréase du produit fini a pu être démontré par ce test. En effet, alors que le pH de la solution témoin augmente, celui contenant la formule de l'exemple 3 reste stable.

### Exemple 11: Activité anti-lipase d'une composition comprenant un extrait total de lupin (sucres de lupin enrichis en galacto-oligosaccharides et peptides de lupin) et un dérivé de chromène ou de chromane

L'activité anti-lipase d'une solution contenant, par rapport au poids total de la solution, 2% en poids d'un extrait total de lupin selon l'invention (sucres de lupin enrichis en galacto-oligosaccharides et peptides de lupin, le rapport massique entre la matière sèche de l'extrait de sucres de lupin et la matière sèche de l'extrait peptidique de lupin étant de 2,5) et 0,1% en poids de génistéine a été évaluée in tubo (modèle biochimique : lipase + substrat + produit à l'essai).

Différentes dilutions ont été testées.

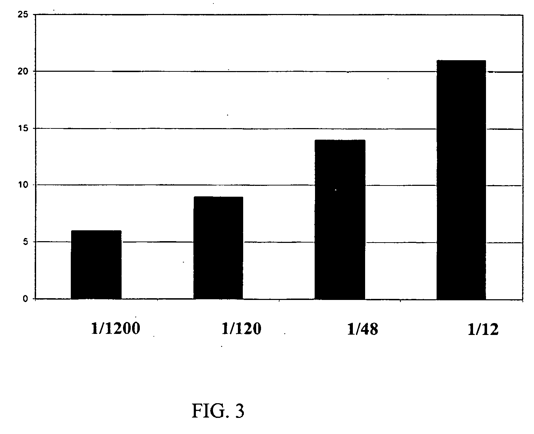

La Figure 3 ci-jointe représente les résultats obtenus (pourcentage d'inhibition de l'activité lipase par rapport au témoin, pour différentes dilutions : 1/1200, 1/120, 1/48 et 1/12). Le mélange extrait total de lupin + génistéine présente une activité anti-lipase hautement significative. Cette activité est dose-dépendante et effective pour des dilutions très faibles (de 1/1200 à 1/12).

### Exemple 12: Test consommateur sous contrôle médical ; évaluation comparative de 3 produits cosmétiques pour le soin de l'épiderme fessier du bébé

Cette étude a été réalisée chez des pédiatres et elle a inclus 127 enfants. L'âge moyen des enfants était de 10,6 mois, 30% d'entres eux avaient une irritation de l'épiderme fessier d''intensité légère et 70% une irritation d'intensité modérée (88% avec une irritation sèche, 12% une irritation suintante).

Après sa première consultation le médecin prescrivait l'une des 2 crèmes du protocole sans en connaître le nom : A= formule de l'exemple 3, B= formule de l'exemple 3 sans principe actif, c'est-à-dire sans extrait total de lupin et sans isoflavone.

Les crèmes étaient appliquées systématiquement après chaque change, pendant 10 à 30 jours. A l'issue de chaque consultation à J0, J 15 et à J30, le praticien réalisait un examen clinique et remplissait un questionnaire. Un questionnaire d'auto-évaluations était également proposé aux utilisateurs (mères).

Les analyses statistiques ont utilisé des tests du Khi2 d'indépendance de chaque variable, des tests non paramétriques de Kruskal et Wallis et de Mann et Whitney.

Les enfants étaient répartis de la manière suivante : A: 87 enfants testés, B : 40 enfants testés.

### a) Disparition des irritations de l'épiderme fessier

L'application après chaque change de la formule A a permis la disparition totale de l'érythème fessier au bout de 4,9 jours chez 85% des bébés testés L'application après chaque change de la formule B a permis la disparition totale de l'érythème fessier au bout de 5,1 jours chez 73% des bébés testés. La formule A a ainsi obtenu le plus fort pourcentage de disparition totale des érythèmes, troubles de la barrière et des irritations de l'épiderme fessier.

Dans les populations restantes l'évolution des lésions étaient favorables mais partielles.

### b) Résultats des questionnaires des auto-évaluations des utilisateurs (mères)

Les performances globales des formules A et B sont indiquées dans le tableau n°6. La comparaison des performances de la formule A à la formule B a permis de confirmer sa supériorité, statistiquement significative, sur la majorité des critères évalués.

**Tableau n°6**

| Critères évalués | | |
|---|---|---|
| **Efficacité** | A | A/B |
| le produit calme les irritations | 92% | S |
| favorise la réparation des lésions | 91% | S |
| réduit les récidives de l'érythème | 80% | S |

| **Agrément cosmétique** | | |
|---|---|---|
| consistance épaisse et couvrante | 97% | S |
| bonne tenue entre deux changes | 94% | S |
| adapté au soin du change bébé | 94% | S |
| niveau de satisfaction pour le produit | 94% | S |

| | | |
|---|---|---|
| S : Critères où la différence constatée entre les 2 produits est statistiquement significative | | |

**Conclusion:** La formule A a permis la disparition rapide et totale des érythèmes et lésions en 4,9 jours dans 85% des cas.

## Revendications

1. Composition comprenant un extrait total de lupin constitué :
- d'un extrait de sucres de lupin, qui comprend au moins 50% en poids, par rapport au poids de la matière sèche de l'extrait de sucres, de galacto-oligosaccharides, et
- d'un extrait peptidique de lupin comprenant au moins 50% en poids de peptides, par rapport au poids total de l'extrait peptidique, susceptible d'être obtenu par un procédé comprenant les étapes suivantes :
i) extraction des lipides de la graine de lupin au moyen d'un solvant approprié et récupération des fractions protéiques et saccharidiques du lupin ;
ii) extraction de la fraction protéique par ultrafiltration et récupération de ladite fraction protéique ;
iii) hydrolyse enzymatique des protéines, récupérées suite à l'étape ii), en peptides ; et
iv) purification par ultrafiltration des peptides obtenus suite à l'étape iii) ;
v) concentration de l'extrait obtenu suite à l'étape iv) par évaporation partielle ou totale de l'eau et récupération de l'extrait peptidique.

2. Composition selon la revendication 1, **caractérisée en ce que** l'extrait de sucres comprend 55 à 90% en poids par rapport au poids de la matière sèche de l'extrait de sucres, de galacto-oligosaccharides.

3. Composition selon la revendication 1 ou 2, **caractérisée en ce que** les galacto-oligosaccharides sont choisis dans le groupe constitué par le verbascose, le stachyose et le raffinose.

4. Composition selon l'une quelconque des revendications précédentes, **caractérisée en ce que** l'extrait de sucres est susceptible d'être obtenu par un procédé comprenant les étapes suivantes :
a) extraction de lipides de la graine de lupin au moyen d'un solvant approprié et récupération des fractions protéiques et saccharidiques du lupin ; puis
b) à partir de la fraction récupérée à l'étape a), séparation de la fraction comprenant les sucres de lupin par ultrafiltration et récupération de ladite fraction saccharidique ; et
c) le cas échéant, raffinage physique de la fraction comprenant les sucres de lupin récupérée suite à l'étape b) ;
d) obtention suite à l'étape b) ou c) d'un extrait de sucres de lupin, très enrichi en galacto-oligosaccharides.

5. Composition selon l'une quelconque des revendications précédentes, **caractérisée en ce que** le rapport massique entre les galacto-oligosaccharides et les peptides de lupin est compris entre 1 et 10, avantageusement entre 2 et 3.

6. Composition selon l'une quelconque des revendications précédentes, **caractérisée en ce que** le lupin est choisi dans le groupe constitué par les variétés *lupinus angustifolius, lupinus albus, lupinus luteus, lupinus mutabilis*, de préférence le lupin est du genre *lupinus albus.*

7. Composition selon l'une quelconque des revendications précédentes, **caractérisée en ce qu'**elle comprend en outre un dérivé de chromane ou de chromène répondant à la formule générale (I) 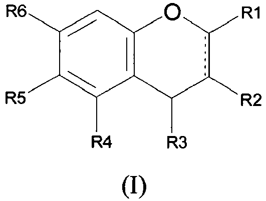 dans laquelle :
la ligne en pointillés représente une liaison additionnelle ou un défaut de liaison additionnelle ;
R₁ représente un atome d'hydrogène, un radical hydroxy, un radical méthoxy, un radical phényle, un radical phényle substitué par 1, 2 ou 3 groupes hydroxy, un radical phényle substitué par 1, 2 ou 3 groupes méthoxy ou un radical phényle substitué par un radical flavone de formule : 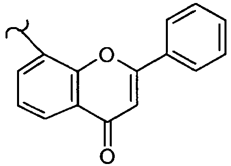
R₂ représente un atome d'hydrogène, un radical hydroxy, un radical méthoxy, un radical phényle, un radical phényle substitué par 1, 2 ou 3 groupes hydroxy ou un radical phényle substitué par 1, 2 ou 3 groupes méthoxy ou bien R₁ et R₂ forment ensemble un cycle benzénique ;
R₃ représente un atome d'hydrogène, un radical hydroxy ou un radical oxo ; R₄, R₅ et R₆, identiques ou différents, représentent un atome d'hydrogène, un radical hydroxy, un radical méthoxy, un radical phényle, un radical phényle substitué par 1, 2 ou 3 groupes hydroxy ou un radical phényle substitué par 1, 2 ou 3 groupes méthoxy.

8. Composition selon la revendication 7, **caractérisée en ce que** le composé (a) est choisi dans le groupe constitué par les chromones, les xanthones et les flavonoïdes.

9. Composition selon la revendication 8, **caractérisée en ce que** le composé (a) est un flavonoïde, avantageusement choisi dans le groupe constitué par les flavones, les flavonols, les dihydro-2,3-flavonols, les flavanones, les flavanols, les flavandiols, les isoflavonoïdes et les biflavonoïdes.

10. Composition selon la revendication 9, **caractérisée en ce que** le flavonoïde est une isoflavone, ou un mélange d'isoflavones, avantageusement choisie dans le groupe constitué par la génistéine, la daidzéine et la glycitéine.

11. Composition cosmétique comprenant une composition selon l'une quelconque des revendications précédentes et un excipient cosmétiquement acceptable.

12. Composition pharmaceutique comprenant une composition selon l'une quelconque des revendications 1 à 10 et un excipient pharmaceutiquement acceptable.

13. Composition comprenant un extrait de sucres de lupin, qui comprend au moins 50 en poids, par rapport au poids de la matière sèche, de galacto-oligosaccharides, à utiliser comme agent anti-inflammatoire.

14. Composition telle que définie à la revendication 12, pour son utilisation dans la prévention et/ou le traitement des érythèmes, en particulier les érythèmes fessiers ou les érythèmes solaires, la couperose, ainsi que toute inflammation cutanée consécutive i) à une agression externe et/ou ii) à un dysfonctionnement du métabolisme ou de la structure de la peau.

15. Méthode de traitement cosmétique des peaux et/ou des muqueuses sensibles, âgées ou présentant un désordre ou un déséquilibre immunologique non pathologique lié au vieillissement intrinsèque, extrinsèque ou hormonal, **caractérisée en ce qu'**elle consiste à appliquer sur la peau et/ou les muqueuses une composition selon la revendication 11.

16. Composition telle que définie à la revendication 12, pour son utilisation pour la prévention et/ou le traitement des peaux et/ou des muqueuses irritées, intolérantes, à tendance allergique ou présentant des rougeurs cutanées.

17. Méthode de traitement cosmétique pour réguler le pH de la peau, **caractérisée en ce qu'**elle consiste à appliquer sur la peau et/ou les muqueuses une composition selon la revendication 11.

18. Composition selon la revendication 16 pour son utilisation par application sur la peau de ladite composition pour réparer l'état de surface de la peau, notamment pour réparer des lésions de la barrière cutanée.

## Patentansprüche

1. Zusammensetzung, umfassend einen Gesamtextrakt aus Lupinen, gebildet aus:
- einem Zuckerextrakt aus Lupinen, der wenigstens 50 Gew.-% im Verhältnis zum Gewicht der Trockenmaterie des Zuckerextrakts, Galakto-Oligosaccharide umfasst
- und einem Peptidextrakt aus Lupinen, umfassend wenigstens 50 Gew.-% Peptide im Verhältnis zum Gesamtgewicht des Peptidextrakts, der durch ein Verfahren erhalten werden kann, das die folgenden Schritte umfasst:
i) Extraktion der Lipide des Lupinenkerns mittels eines geeigneten Lösungsmittels und Auffangen der Protein- und Saccharinfraktionen der Lupine:
ii) Extraktion der Proteinfraktion per Ultrafiltern und Auffangen der genannten Proteinfraktion;
iii) Enzymatische Hydrolyse der Proteine, die im Anschluss an den Schritt ii) aufgefangen wurden, in Peptide; und
iv) Reinigung der Peptide per Ultrafilterung, die im Anschluss an den Schritt iii) erhalten wurden;
v) Konzentration des erhaltenen Extraktes im Anschluss an den Schritt iv) per teilweisem oder totalem Verdampfen des Wassers und Auffangen des Peptidextraktes.

2. Zusammensetzung gemäß Anspruch 1, **dadurch gekennzeichnet, dass** der Zuckerextrakt 55 bis 90 Gew.-% im Verhältnis zum Gewicht der Trockenmaterie des Zuckerextrakts an Galakto-Oligosacchariden umfasst.

3. Zusammensetzung gemäß Anspruch 1 oder 2, **dadurch gekennzeichnet, dass** die Galakto-Oligosaccharide aus der Gruppe ausgewählt sind, bestehend aus Verbaskose, Stachyose und Raffinose.

4. Zusammensetzung gemäß irgendeinem der voranstehenden Ansprüche, **dadurch gekennzeichnet, dass** der Zuckerextrakt geeignet ist, durch ein Verfahren erhalten zu werden, umfassend die folgenden Schritte:
a) Extraktion von Lipiden des Lupinenkerns mittels eines geeigneten Lösungsmittels und Auffangen der Protein- und Saccharidfraktionen der Lupine; dann
b) ausgehend von der in Schritt a) aufgefangenen Fraktion Trennung der Fraktion, umfassend den Lupinenzucker per Ultrafilterung und Auffangen der genannten Saccharidfraktion; und
c) ggf. physisches Raffinieren der Fraktion, umfassend den im Anschluss an den Schritt b) aufgefangenen Lupinenzucker;
d) im Anschluss an den Schritt b) oder d) Erhalt eines Zuckerextrakts aus Lupinen, der stark mit Galacto-Oligosacchariden angereichert ist.

5. Zusammensetzung gemäß einem der voranstehenden Ansprüche, **dadurch gekennzeichnet, dass** das Masseverhältnis zwischen den Galacto-Oligosacchariden und den Lupinenpeptiden zwischen 1 und 10, vorteilhaft zwischen 2 und 3, inbegriffen ist.

6. Zusammensetzung gemäß einem der voranstehenden Ansprüche, **dadurch gekennzeichnet, dass** die Lupine aus der Gruppe ausgewählt ist, gebildet aus den Sorten *lupinus angustifolius, lupinus albus, lupinus luteus, lupinus mutabilis,* bevorzugt ist die Lupine von der Art *lupinus albus.*

7. Zusammensetzung gemäß einem der voranstehenden Ansprüche, **dadurch gekennzeichnet, dass** sie darüber hinaus ein Derivat aus Chroman oder Chromen umfasst, das der allgemeinen Formel (I) entspricht: 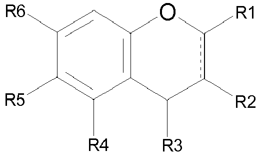 in der:
die gestrichelte Linie eine zusätzliche Verbindung oder eine fehlende zusätzliche Verbindung darstellt;
R₁ ein Wasserstoffatom, ein Hydroxylradikal, ein Methoxylradikal, ein Phenylradikal, ein durch 1, 2 oder 3 Hydroxylgruppen substituiertes Phenylradikal, ein durch 1, 2 oder 3 Methoxylgruppen substituiertes Phenylradikal oder ein durch ein Flavonradikal mit der folgenden Formel substituiertes Phenylradikal darstellt: 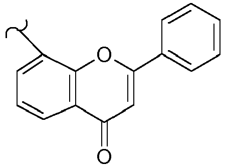
R₂ ein Waserstoffatom, ein Hydroxylradikal, ein Methoxylradikal, ein Phenylradikal, ein durch 1, 2 oder 3 Hydroxylgruppen substituiertes Phenylradikal oder ein durch 1, 2 oder 3 Methoxylgruppen substituiertes Phenylradikal darstellt oder R₁ und R₂ zusammen einen Benzolzyklus bilden;
R₃ ein Wasserstoffatom, ein Hydroxylradikal oder ein Oxoradikal darstellt;
R₄, R₅ und R₆, die identisch oder unterschiedlich sind, ein Wasserstoffatom, ein Hydroxylradikal, ein Methoxylradikal, ein Phenylradikal, ein durch 1, 2 oder 3 Hydroxylgruppen substituiertes Phenylradikal oder ein durch 1, 2 oder 3 Methoxylgruppen substituiertes Phenylradikal darstellen.

8. Zusammensetzung gemäß Anspruch 7, **dadurch gekennzeichnet, dass** die Verbindung (a) aus der Gruppe ausgewählt ist, gebildet aus Chromonen, Xanthonen und Flavonoiden.

9. Zusammensetzung gemäß Anspruch 8, **dadurch gekennzeichnet, dass** die Verbindung (a) in Flavonoid ist, das vorteilhaft aus der Gruppe ausgewählt ist, gebildet aus Flavonen, Flavonolen, Dihydro-2,3-Flavonolen, Flavononen, Flavanolen, Flavandiolen, Isoflavonoiden und Biflavonoiden.

10. Zusammensetzung gemäß Anspruch 9, **dadurch gekennzeichnet, dass** das Flavonoid ein Isoflavon oder eine Mischung aus Isoflavonen ist, die vorteilhaft aus der Gruppe ausgewählt ist, gebildet aus Genistein, Daidizein und Glycitein.

11. Kosmetische Zusammensetzung, umfassend eine Zusammensetzung gemäß einem der voranstehenden Ansprüche und einem kosmetisch akzeptablen Trägerstoff.

12. Pharmazeutische Zusammensetzung, umfassend eine Zusammensetzung gemäß irgendeinem der Ansprüche 1 bis 10, und einem pharmazeutisch akzeptablen Trägerstoff.

13. Zusammensetzung, umfassend einen Zuckerextrakt aus Lupinen, der wenigstens 50 Gew.-% im Verhältnis zum Gewicht der Trockenmaterie an Galakto-Oligosacchariden umfasst, die als entzündungshemmender Wirkstoff zu verwenden sind.

14. Zusammensetzung gemäß Definition in Anspruch 12 für ihre Verwendung bei der Prävention und / oder der Behandlung von Erythemen, insbesondere Erythemen des Gesäßes oder Sonnenerytheme, Kuperose sowie jeglicher Hautentzündung im Anschluss an i) eine externe Aggression und / oder ii) eine Störung des Stoffwechsels oder der Struktur der Haut.

15. Kosmetisches Behandlungsverfahren der Haut und / oder der sensiblen, gealterten oder der Schleimhäute, die eine Störung oder ein immunologisches, nicht pathologisches Ungleichgewicht im Zusammenhang mit einer intrinsischen, extrinsischen oder hormonalen Alterung aufweisen, **dadurch gekennzeichnet, dass** sie in der Anwendung einer Zusammensetzung gemäß Anspruch 11 auf die Haut und / oder die Schleimhäute besteht.

16. Zusammensetzung gemäß Definition in Anspruch 12 für ihre Verwendung zur Prävention und / oder Behandlung der Haut und / oder gereizter, intoleranter Schleimhäute mit allergischer Tendenz oder die Hautrötungen aufweisen.

17. Kosmetisches Behandlungsverfahren zur Regulierung des pH-Wertes der Haut, **dadurch gekennzeichnet, dass** es in der Anwendung einer Zusammensetzung gemäß Anspruch 11 auf die Haut und / oder die Schleimhäute besteht.

18. Zusammensetzung gemäß Anspruch 16 für die Verwendung der genannten Zusammensetzung per Anwendung auf die Haut zum Heilen des Oberflächenzustandes der Haut, insbesondere zum Heilen von Schäden der Hautbarriere.

## Claims

1. Composition comprising a total extract of lupin constituted:
- of an extract of sugars of lupin, which comprises at least 50% by weight, with respect to the weight of the dry matter of the extract of sugars, of galacto-oligosaccharides, and
- of an peptide extract of lupin comprising at least 50% by weight of peptides, with respect to the total weight of the peptide extract, able to be obtained by a method comprising the following steps:
i) extracting lips from the grain of lupin by means of a suitable solvent and recovering protein and saccharide fractions of the lupin;
ii) extracting the protein fraction via ultrafiltration and recovering said protein fraction;
iii) enzymatic hydrolysis of proteins, recovered following step ii), into peptides; and
iv) purifying via ultrafiltration of the peptides obtained following the step iii);
v) concentrating the extract obtained following the step iv) by partial or total evaporation of the water and recovering the peptide extract.

2. Composition according to claim 1, **characterised in that** the extract of sugars comprises 55 to 90% by weight with respect to the weight of the dry matter of the extract of sugars, of galacto-oligosaccharides.

3. Composition according to claim 1 or 2, **characterised in that** the galacto-oligosaccharides are chosen from the group comprising verbascose, stachyose and raffinose.

4. Composition as claimed in any preceding claim, **characterised in that** the extract of sugars is able to be obtained by a method comprising the following steps:
a) extracting of lips from the grain of lupin my means of a suitable solvent and recovering of the protein and saccharide fractions of the lupin; then
b) using the fraction recovered in the step a), separating the fraction comprising the sugars of lupin via ultrafiltration and recovering of said saccharide fraction; and
c) where applicable, physical refining of the fraction comprising the sugars of lupin recovered following the step b);
d) obtaining following the step b) or c) of an extract of sugars of lupin, highly enriched with galacto-oligosaccharides.

5. Composition as claimed in any preceding claim, **characterised in that** the mass ratio between the galacto-oligosaccharides and the peptides of lupin is between 1 and 10, advantageously between 2 and 3.

6. Composition as claimed in any preceding claim, **characterised in that** the lupin is chosen from the group comprising the varieties *lupinus angustifolius, lupinus albus, lupinus luteus, lupinus mutabilis,* more preferably the lupin is of the genus *lupinus albus.*

7. Composition as claimed in any preceding claim, **characterised in that** it further comprises a derivative of chroman or of chromene satisfying the general formula (I) 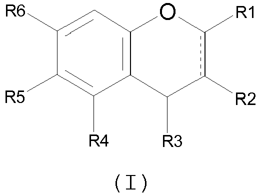 wherein:
the dotted line shows an additional bond or a lack of additional bond;
R₁ represents an atom of hydrogen, a hydroxy radical, a methoxy radical, a phenyl radical, a phenyl radical substituted with 1, 2 or 3 hydroxy groups, a phenyl radical substituted by 1, 2 or 3 methoxy groups or a phenyl radical substituted by a flavone radical of formula: 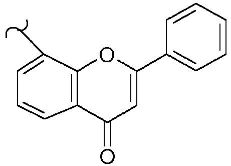
R₂ represents an atom of hydrogen, a hydroxy radical, a methoxy radical, a phenyl radical, a phenyl radical substituted with 1, 2 or 3 hydroxy groups or a phenyl radical substituted by 1, 2 or 3 methoxy groups
or R₁ and R₂ form together a benzenic cycle;
R₃ represents an atom of hydrogen, a hydroxy radical or an oxo radical;
R₄, R₅ and R₆, identical or different, represent an atom of hydrogen, a hydroxy radical, a methoxy radical, a phenyl radical, a phenyl radical substituted by 1, 2 or 3 hydroxy groups or a phenyl radical substituted by 1, 2 or 3 methoxy groups.

8. Composition according to claim 7, **characterised in that** the compound (a) is chosen from the group constituted by chromones, xanthones and flavonoids.

9. Composition according to claim 8, **characterised in that** the compound (a) is a flavonoid, advantageously chosen from the group constituted by flavones, flavonols, dihydro-2,3-flavonols, flavanones, flavanols, flavandiols, isoflavonoids and biflavonoids.

10. Composition according to claim 9, **characterised in that** the flavonoid is a isoflavone, or a mixture of isoflavones, advantageously chosen from the group constituted by genistein, daidzein and glycitein.

11. Cosmetic composition comprising a composition as claimed in any preceding claim and a cosmetically acceptable excipient.

12. Pharmaceutical composition comprising a composition according to any of claims 1 to 10 and a pharmaceutically acceptable excipient.

13. Composition comprising an extract of sugars of lupin, which comprises at least 50% by weight, with respect to the weight of the dry matter, of galacto-oligosaccharides, to be used as an anti-inflammatory agent.

14. Composition such as defined in claim 12, for its use in the prevention and/or the treatment of rashes, in particular buttock rashes or solar rashes, rosacea, as well as any skin inflammation consecutive i) to an external aggression and/or ii) a malfunction of the metabolism or of the structure of the skin.

15. Method for the cosmetic treatment of the skin and/or of sensitive mucosa, aged or having a disorder or a non-pathological immunological imbalance linked to intrinsic, extrinsic or hormonal ageing, **characterised in that** it consists in applying on the skin and/or the mucosa a composition according to claim 11.

16. Composition such as defined in claim 12, for its use in the prevention and/or the treatment of the skin and/or of irritated, intolerant mucosa, with allergic trends or having skin redness.

17. Method for the cosmetic treatment for adjusting the pH of the skin, **characterised in that** it consists in applying on the skin and/or the mucosa a composition according to claim 11.

18. Composition according to claim 16 for its use by application on the skin of said composition for repairing the surface condition of the skin, in particular for repairing lesions of the skin barrier.
